# EUROPEAN PATENT APPLICATION

(11) **EP 4 375 654 A1**
(43) Date of publication of application: **29.05.2024**
(21) Application number: 22845826.1
(22) Date of filing: 13.07.2022
(51) Int. Cl.: G01N 27/02, G01N 33/483, G01N 33/53, G01N 33/569

(54) **METHOD AND DEVICE FOR DETECTING BIOMOLECULE**

(30) Priority: 18.07.2021 JP 2021118354
(71) Applicant: The University of Tokyo, Bunkyo-ku, Tokyo 113-8654 (JP); Provigate Inc., Tokyo 113-0033 (JP)
(72) Inventor: SAKATA Toshiya, Tokyo 113-8654 (JP); NISHITANI Shoichi, Tokyo 113-8654 (JP); SHIRATORI Reiko, Tokyo 113-8654 (JP); SEKIMIZU Koshin, Tokyo 113-0033 (JP); ITO Narushi, Tokyo 113-0033 (JP)
(74) Representative: Michalski Hüttermann & Partner Patentanwälte mbB
(86) International application number: PCT/JP2022/027516
(87) International publication number: WO 2023/002892

(57) **Abstract**

The present disclosure provides a method for detecting a biomolecule. The method includes providing an electrode 101 and an aptamer 102 that specifically binds to a target biomolecule and has a charge, the aptamer 102 being disposed near the electrode 101; introducing a cationic mediator 113 having a charge opposite to the charge of the aptamer 102 to the electrode 101 at which the aptamer 102 is disposed; bringing a solution containing the target biomolecule into contact with the aptamer 102 to cause the aptamer 102 to bind to the biomolecule; and measuring an electrical signal that is produced at the electrode 101 in association with the cationic mediator 113.

## Description

### FIELD OF INVENTION

The present disclosure relates to a method and an apparatus for detecting a biomolecule.

### BACKGROUND ART

In biosensing, there is a need for technologies for more accurately and/or more conveniently determining the amount or the presence or absence of a biomolecule present in a solution, such as a body fluid. They are also effective for point-of-care testing. They are also desired for the testing of a trace amount of an analyte.

### SUMMARY OF THE INVENTION

For example and without limitation, recognized herein is a need to more accurately recognize a biomolecule that is to be measured, among various substances (e.g., proteins) present in a body fluid, and to measure the biomolecule.

Some embodiments of the present disclosure provide a method for detecting a biomolecule. In some embodiments, the method includes providing an electrode and an aptamer that is disposed near the electrode and which specifically binds to a target biomolecule. In some embodiments, the method includes introducing a cationic mediator to the electrode at which the aptamer is disposed. In some embodiments, the method includes bringing a solution containing the target biomolecule into contact with the aptamer to cause the aptamer to bind to the biomolecule. In some embodiments, the method includes measuring an electrical signal that is produced at the electrode in association with the cationic mediator.

With the embodiments described above, it is possible to accurately quantify, for example, an amount of a biomolecule present in a body fluid.

Additional aspects and advantages of the present disclosure will become readily apparent to those skilled in this art from the following detailed description, wherein only illustrative embodiments of the present disclosure are shown and described. As will be understood, the present disclosure is capable of other and different embodiments, and modifications can be made to some of its details in various obvious respects without departing from the present disclosure. Accordingly, the drawings and description are to be regarded as illustrative in nature, and not as restrictive.

### BRIEF DESCRIPTION OF DRAWINGS

[Fig. 1A] Fig. 1A is a schematic cross-sectional view of a biomolecule detection device according to an embodiment.
[Fig. 1B] Fig. 1B is a schematic cross-sectional view of a biomolecule detection device according to an embodiment.
[Fig. 1C] Fig. 1C is a schematic cross-sectional view of a biomolecule detection device according to an embodiment.
[Fig. 2A] Fig. 2Ais a schematic cross-sectional view of a biomolecule detection device according to an embodiment.
[Fig. 2B] Fig. 2B is a schematic cross-sectional view of a biomolecule detection device according to an embodiment.
[Fig. 3] Fig. 3 is a schematic cross-sectional view of a biomolecule detection device according to an embodiment.
[Fig. 4A] Fig. 4Ais a Nyquist plot of electrochemical impedance spectroscopy obtained in a measurement according to an embodiment.
[Fig. 4B] Fig. 4B is a Nyquist plot of electrochemical impedance spectroscopy obtained in a measurement according to an embodiment.
[Fig. 5] Fig. 5 is a graph illustrating a relationship between a detection output and a concentration, obtained from the measurement results shown in Figs. 4A and 4B.
[Fig. 6] Fig. 6 is a schematic cross-sectional view of a biomolecule detection device according to an embodiment.
[Fig. 7] Fig. 7 is a schematic cross-sectional view of a biomolecule detection device according to an embodiment.
[Fig. 8A] Fig. 8A is a Nyquist plot of electrochemical impedance spectroscopy obtained in a measurement according to an embodiment.
[Fig. 8B] Fig. 8B is a Nyquist plot of electrochemical impedance spectroscopy obtained in a measurement according to an embodiment.
[Fig. 8C] Fig. 8C is a Nyquist plot of electrochemical impedance spectroscopy according to an embodiment.
[Fig. 9] Fig. 9 is a graph illustrating a relationship between a detection output and a concentration, obtained from measurement results according to an embodiment.
[Fig. 10A] Fig. 10A is a schematic cross-sectional view of a biomolecule detection device according to an embodiment.
[Fig. 10B] Fig. 10B is a schematic cross-sectional view of a biomolecule detection device according to an embodiment.
[Fig. 11A] Fig. 11A is a schematic cross-sectional view of a biomolecule detection device according to an embodiment.
[Fig. 11B] Fig. 11B is a schematic cross-sectional view of a biomolecule detection device according to an embodiment.
[Fig. 12A] Fig. 12A is a Nyquist plot of electrochemical impedance spectroscopy obtained in a measurement according to an embodiment.
[Fig. 12B] Fig. 12B is a Nyquist plot of electrochemical impedance spectroscopy obtained in a measurement according to an embodiment.
[Fig. 13] Fig. 13 is a graph illustrating a relationship between a detection output and a concentration, obtained from measurement results according to an embodiment.
[Fig. 14] Fig. 14 is a flowchart of a method for measuring a biomolecule according to an embodiment.
[Fig. 15] Fig. 15 is a flowchart of a method for measuring a biomolecule according to an embodiment.

### DESCRIPTION OF EMBODIMENTS

As used herein, the term "biomolecule" generally refers to a biological molecule or a molecule that is present or functions in vivo. Biomolecules include, for example and without limitation, proteins, oligonucleotides, nucleic acids (DNAs and RNAs), amino acids, peptides, lipids, cells, vesicles, sugars, carbohydrates, antibodies, and modified or altered products thereof.

Proteins as target biomolecules include, for example and without limitation, albumin and hemoglobin. In some embodiments, the target biomolecule may be glucose. Other target biomolecules include, for example and without limitation, neurotransmitters, such as dopamine, metabolites, diabetes markers, cancer markers, allergy-associated substances, such as histamine, and Alzheimer's-associated substances, such as amyloid β.

Biomolecules according to the present disclosure include, for example and without limitation, biomolecules such as bacteria- or virus-derived nucleic acids and proteins. The viruses include, for example and without limitation, influenza vivirusronaviruses, noroviruses, and Ebola viruses. In some embodiments, the biomolecules include extracellular vesicles. The extracellular vesicles (EVs) may be exosomes.

In some embodiments, the target biomolecules include glycated biomolecules. In some embodiments, the target biomolecule may be a glycated protein. In some embodiments, the target biomolecule may be glycated albumin.

The target biomolecules include, for example and without limitation, glycated hemoglobin (HbA1c); glycated proteins containing sialic acid, which is frequently found on cancer cell surfaces, and associated glycopeptides; and advanced glycation end-products (AGEs), which include Nε-(carboxymethyl)lysine (CML), Nε-(carboxyethyl)lysine (CEL), argpyrimidine, pentosidine, pyraline, crossline, GA-pyridine, Nco-(carboxymethyl)arginine (CMA), furoyl-furanyl imidazole, and glucosepane.

In some embodiments, the target biomolecule may include a plurality of types of target biomolecules.

The biomolecules may be naturally occurring biomolecules or artificially produced biomolecules.

In some embodiments, the target biomolecule may be provided in a liquid (solution). The liquid may be a body fluid secreted by a subject or may be a liquid other than a body fluid. The liquid other than a body fluid may be a liquid present on a subject or a liquid that is not present on a subject. The liquid that is not present on a subject may be a liquid held in a subject.

The liquid to be provided may contain a target biomolecule, may have a possibility of containing a target biomolecule, or may be a standard solution that is used for the measurement of a target biomolecule. The liquids, including the liquids of these cases, may hereinafter be referred to as liquids (or solutions) containing a target biomolecule.

The liquid containing a target biomolecule may be a solution. The liquid may be a body fluid, a solution that is body-fluid-derived, or a diluted solution of a body fluid. The liquid may be a solution that is not a body fluid (not body-fluid-derived) or may be a liquid mixture of a body fluid or a solution that is body-fluid-derived and a solution that is not body-fluid-derived. The solution may be a solution for use in a measurement of samples or a solution for use in a measurement for calibration. For example, the solution may be a standard solution or a calibration solution. The sample that is a measurement target may be an analyte.

The body fluid may be a lymph fluid, may be a tissue fluid, such as an intercellular fluid, a transcellular fluid, or an interstitial fluid, or may be a body cavity fluid, a serous cavity fluid, a pleural fluid, an ascites fluid, a pericardial fluid, a cerebrospinal fluid (spinal fluid), a joint fluid (synovial fluid), or an eye's aqueous humor (aqueous humor). The body fluid may be a digestive fluid, such as saliva, gastric juice, bile, pancreatic juice, or an intestinal fluid, or may be sweat, tears, nasal mucus, urine, semen, a vaginal fluid, an amniotic fluid, or milk. The body fluid may be an animal body fluid or a human body fluid. "Body fluid" may be a solution. The solution may include a physiological buffer containing the measurement target substance. Examples of the physiological buffer include phosphate buffered saline (PBS), N-tris(hydroxymethyl)methyl-2-aminoethanesulfonic acid buffers (TES), and hydroxyethyl piperazine ethanesulfonic acid buffers (HEPES). The solution is not particularly limited as long as the solution contains the measurement target substance.

In some embodiments, the body fluid may be blood. In some embodiments, blood may be collected. For example, blood may be collected upon the occurrence of bleeding due to puncture. For example, blood may be sucked through a needle that has been inserted. In some embodiments, a puncturing tool (e.g., a needle or an injection needle; the same applies hereinafter) may be disposed at an end of a capillary tube. In some embodiments, a capillary tube may be formed as a puncturing tool.

In some embodiments, the subject may include a human or may be a human. In some embodiments, the subject may include a non-human animal or may be a non-human animal. The non-human animal may include a mammalian animal or may be a mammalian animal. For example and without limitation, the non-human animal may be a working animal, a livestock animal, a pet animal, or a wild animal.

As used herein, the term "electrode" is a broad term, and its ordinary and customary meaning should be given to those of ordinary skill in the art (the term should not be limited to particular or special meanings). In some embodiments, an electrode may be used in an electrochemical measurement. The electrode may be formed of a conductive material. The electrode may be formed of a metal. For example and without limitation, the electrode may be formed with a metal material, such as gold (Au), platinum (Pt), or palladium (Pd). The electrode may be formed of a non-metallic material. For example and without limitation, the electrode may be formed of carbon (C). The electrode may be formed of graphene, a carbon nanotube, or the like.

The electrode may be connected to, or be configured to be connected to, a measurement instrument, a measurement device, or a measurement element.

In some embodiments, the electrode may be connected to an impedance measurement instrument. In some embodiments, an electrical signal at the electrode may be detected or analyzed by electrochemical impedance spectroscopy (EIS). A Cole-Cole plot or a Nyquist plot may be generated accordingly. A capacitance component of the measurement system may be determined or analyzed. In some embodiments, the electrical signal at the electrode may be detected or analyzed by cyclic voltammetry (CV).

In some embodiments, an electrode located near an aptamer may be used as a working electrode (WE). In addition, a counter electrode (CE) and a reference electrode (RE) may be disposed.

In some embodiments, the electrode may be connected to a current measurement instrument, an ammeter, an amperometer, or the like. The electrical signal at the electrode may be measured or analyzed amperometrically.

In some embodiments, the electrode may be connected to a voltmeter, a potentiometer, a transistor (e.g., a field effect transistor), or the like. The electrical signal at the electrode may be measured or analyzed potentiometrically.

In some embodiments, a device or an apparatus may include multiple electrodes. In some embodiments, the multiple electrodes may have the same aptamer. In some embodiments, at least some or all of the multiple electrodes have aptamers different from one another. In this case, for example, different biomolecules can be detected or measured on a single device. In some embodiments, at least some or all of the multiple electrodes have aptamers having densities different from one another. In this case, for example, proper measurements can be carried out for different respective concentration ranges. In some embodiments, the device or apparatus may include multiple electrodes arranged in an array. In this case, for example, integration of a sensor can be achieved. Additionally or alternatively, for example, regarding a reaction with the introduced biomolecule, its aptamer, or the like, it is possible to measure or observe a two-dimensional or spatial distribution or its temporal changes.

In some embodiments, the device or apparatus may include multiple electrodes, with some of the electrodes having a different sensing configuration that does not involve the use of an aptamer. In some embodiments, the device or apparatus may include multiple electrodes, with some of the electrodes having an aptamer and with some or all of the other electrodes having a different sensing configuration. For example, some of the multiple electrodes may have a sensing configuration that recognizes a biomolecule different from the target biomolecule of the aptamer. For example, these electrodes may have an antibody that recognizes a specific biomolecule.

As used herein, the term "aptamer" is used interchangeably with the term "nucleic acid ligand" and refers to a DNA, RNA, oligonucleotide, or peptide molecule that binds to a specific target molecule. The aptamer may be single-stranded. In general, aptamers are relatively inexpensive and have a long life.

In some embodiments, the aptamer may be disposed near an electrode. In some embodiments, the aptamer may be anchored to the electrode. The aptamer may be directly anchored to the electrode or may be indirectly anchored to the electrode with a different substance disposed therebetween. In some embodiments, the aptamer need not be anchored to the electrode. In some embodiments, an anchor layer may be formed on a surface of the electrode, and the aptamer may be anchored to the anchor layer or may be synthesized with the anchor layer serving as a scaffold. In some embodiments, the anchor layer may be formed as a protective film that is as described in the present disclosure. That is, the anchor layer and the protective film may be defined as being the same member. In some embodiments, the anchor layer may be disposed in addition to the protective film, and the anchor layer may be formed on an upper side of the protective film (on the opposite side from the electrode) or on a lower side of the protective film (between the electrode and the protective film), as viewed from the electrode.

In some embodiments, the aptamer may be an aptamer that specifically binds to a protein. The aptamer may be single-stranded. In some embodiments, the aptamer may be an aptamer that specifically binds to glycated albumin. A nucleotide sequence that forms the aptamer may be as follows (Table 1).
5'-TGCGGTTGTAGTACTCGTGGCCG-3' (SEQ ID NO: 1)

In some embodiments, the biomolecule to which the aptamer specifically binds may be a protein expressed on a cell surface or may be a membrane protein on a cell surface. In some embodiments, the aptamer may be an aptamer that specifically binds to a membrane protein on a surface of a leukemia cancer cell, and a nucleotide sequence that forms the aptamer may be as follows (Table 1).

5'-
AAAAAAAAAAATCTAACTGCTGCGCCGCCGGGAAAATACTGTACGGTTAGA-3'
(SEQ ID NO:2)

In some embodiments, the aptamer may be an aptamer that specifically binds to a virus. For example and without limitation, the virus may be a coronavirus. For example and without limitation, the coronavirus may be a SARS virus. The coronavirus may be a SARS-C coronavirus OVID-19 virus. A nucleotide sequence that forms the aptamer may be as follows (Table 1).

5'-
GCAATGGTACGGTACTTCCGGATGCGGAAACTGGCTAATTGGTGAGGCTGGGGCG
GT-3' (SEQ ID NO:3)

5'-
CAGCACCGACCTTGTGCTTTGGGAGTGCTGGTCCAAGGGCGTTAATGGACA-3'
(SEQ ID NO:4)

5'-

ATCCAGAGTGACGCAGCATTTCATCGGGTCCAAAAGGGGCTGCTCGGGATTGCGG

ATATGGACACGT-3' (SEQ ID NO:5)

The nucleotide sequence of the aptamer is not limited to one or more of the nucleotide sequences selected from those set forth above in SEQ ID NO: 1 to SEQ ID NO:5. The aptamer may include another nucleotide sequence or may be an aptamer that recognizes another biomolecule.
[Table 1]
*

"Mediator" as used herein may have a charge opposite to that of the aptamer. "Cationic mediator" as used herein refers to a mediator that has a positive charge or is positively charged and which can be used in an electrochemical measurement. The nucleic acid that forms an aptamer generally has a negative charge, for example. The cationic mediator may be a complex having a cationic ligand. The cationic mediator may be, for example, a ruthenium complex (also referred to as a "complex ion"). In some embodiments, the ruthenium complex may be a ruthenium (II) complex. The ruthenium complex may be, for example, [Ru(NH₃)₆]²⁺. The cationic mediator may be, for example, an osmium complex. In some embodiments, the osmium complex may be an osmium (II) complex. In some embodiments, the osmium complex may be a cobalt (II) complex ([Co(bpy)₃]³⁺/²⁺, [Co(phen)₃]³⁺/²⁺, or the like).

The mediator may be provided in a solution. For example, the mediator may be dissolved or included in water. For example and without limitation, the solution for the mediator may be a buffer, such as phosphate buffered saline (PBS).

A protective film or a protective layer (these terms have the same meaning or are used interchangeably herein) may be disposed on a surface of the electrode. The protective film may have an ability to substantially prevent a substance (foreign material or the like) that has an influence on the measurement of the target substance from reaching, coming into contact with, approaching, and/or adsorbing to the electrode, thereby substantially exerting a chemical or electrical influence on the electrode; or the protective film may have an ability to reduce the influence.

The protective film may be formed of an organic material, an inorganic material, or a mixture thereof. In some embodiments, the protective film may be formed substantially of a polymer.

The protective film may have an ability to allow the mediator to pass therethrough to an extent that the measurement with the electrode can be sufficiently performed. The protective film may have an ability to prohibit or limit the passage of foreign materials therethrough.

In some embodiments, the protective film may be an aryl-based single-layer film or multi-layer film. The aryl-based film may be formed by electrochemical grafting. A diazonium molecule may be used. The diazonium molecule or electronic diazonium molecule may be, for example, a 4-nitrobenzene diazonium salt. The aryl-based film may be formed by cyclic voltammetry (CV).

The aryl-based film may be formed by using a radical scavenger (which is also referred to as a radical eliminator or a radical remover). For example and without limitation, the radical scavenger may be 2,2-diphenyl-1-picrylhydrazyl (DPPH). A single-layer film may be formed by using a radical scavenger. Diazonium radicals that are electrochemically generated from diazonium molecules bind to a surface of the electrode (e.g., Au) in the presence of a radical scavenger. This enables the formation of a relatively dense film. The higher the proportion of coverage of the surface by the grafted monolayer, the greater the extent to which a redox reaction between the molecules and the surface of the electrode is inhibited. For example, the monolayer may be formed with a density that allows the mediator to pass therethrough.

A multi-layer film can be formed, for example, by repeatedly performing electrochemical polymerization by cyclic voltammetry (CV) on a diazonium layer that has been grafted as a single-layer film. The diazonium radicals that have been electricitically generated do not react with the surface of the electrode unless there is a radical scavenger or the like but react with the grafted diazonium. Accordingly, a multi-layer film can be formed by diazonium grafting without changing the density of the initial diazonium layer on the surface of the electrode. Thus, the multi-layer film has some gaps. It is also possible to control a thickness of the multi-layer film. Thus, foreign materials are inhibited from approaching the surface of the electrode, and biomolecules with a relatively low molecular weight can be allowed to reach the surface of the electrode through the gaps of the multi-layer film.

### <First Embodiment>

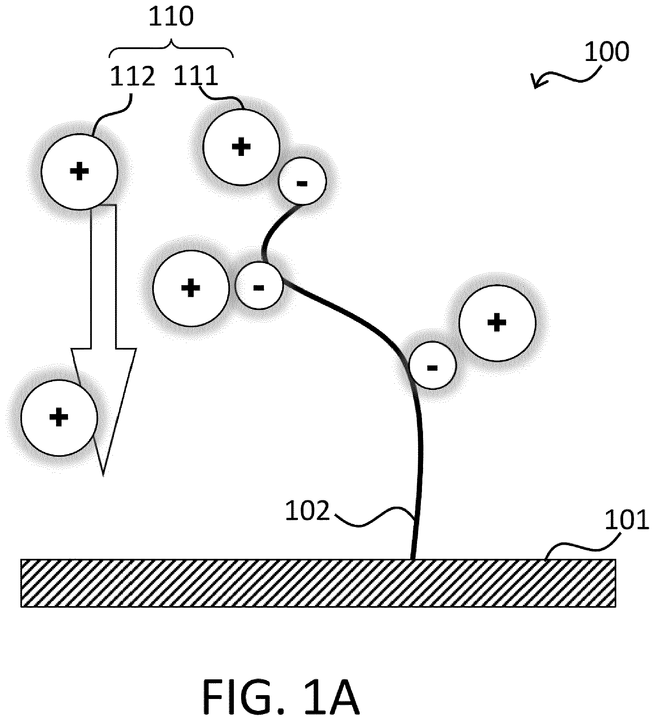

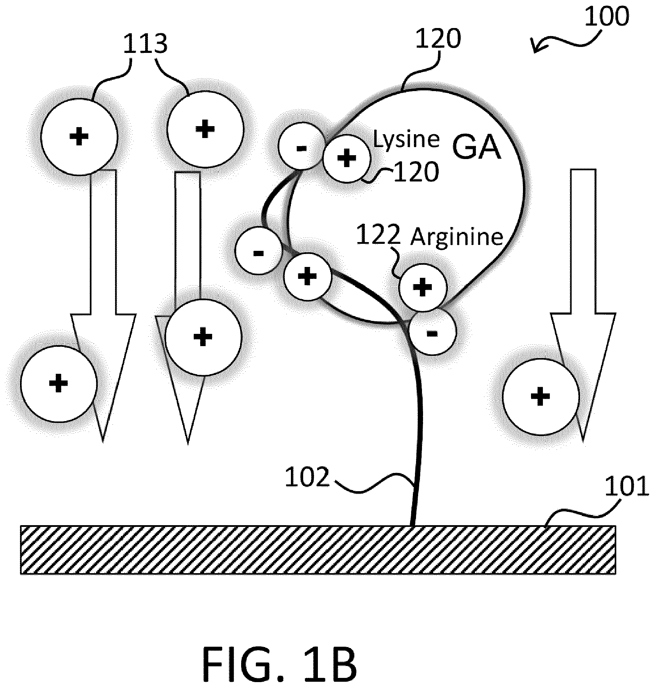

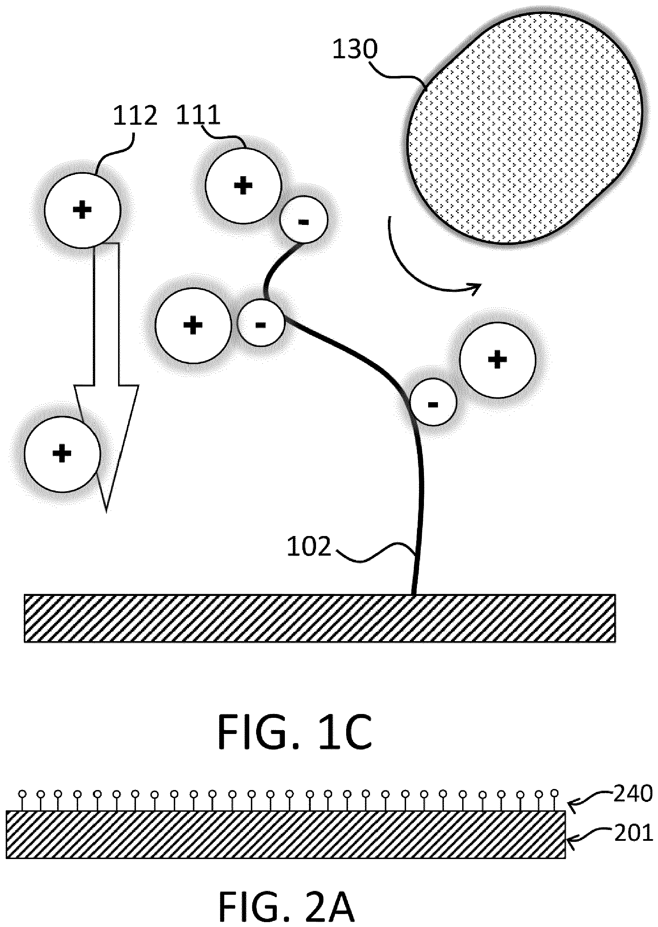

An electrochemical sensor according to an embodiment and a method for measuring a biomolecule by using the electrochemical sensor will be described with reference to Fig. 1. Figs. 1A and 1B schematically illustrate basic configurations of an electrochemical sensor 100, associated respectively with an instance in which no target biomolecule is present in the system and an instance in which a target biomolecule is present in the system. Figs. 1 to 1C illustrate an example in which the target biomolecule used is glycated albumin (GA), and the aptamer used is a nucleic acid ligand that specifically binds to glycated albumin, that is, an aptamer having a nucleotide sequence of SEQ ID NO: 1. The foreign material used is human serum albumin (HSA) or an immunoglobulin (e.g., IgG). These are examples and should not be construed as limiting the present disclosure.

The sensor 100 illustrated in Fig. 1A includes an electrode 101 and an aptamer 102 which is disposed on the electrode 101 and has a negative charge. Sensors such as the sensor 100 can be produced, for example, in the following manner. First, a multi-layer film or a single-layer film is obtained by electrochemically anchoring or electrodepositing an aryl diazonium salt on a surface of the electrode. Next, N-succinimidyl 3-maleimidobenzoate (MBS) (having a carboxyl group and a maleimide group at both ends) is reacted with a functional group (e.g., an amino group) present in the multi-layer film or the single-layer film. This causes a reaction between the carboxyl group and the amino group present in the multi-layer film or the single-layer film. Accordingly, the maleimide terminal becomes a free end. A thiolated DNA (aptamer) is reacted with the free end. This causes the aptamer molecule to be anchored to the surface of the electrode.

In the environment in which no target substance is present, when a cationic mediator 110 is introduced, some portions of the cationic mediator, which are referred to as a cationic mediator 111, are attracted to sites of the aptamer 102 that have an electrically opposite charge. Other portions of the cationic mediator, which are referred to as a cationic mediator 112, reach the electrode 101 without feeling the aptamer 102, which has been rendered electrically neutral. The cationic mediator 112 that has reached the electrode 101 is detected as an electrical signal at the electrode 101.

Referring to Figs. 1A to 1C, the cationic mediators 110, 111, and 112 may be a ruthenium complex [Ru(NH₃)₆]²⁺.

At the electrode 101, the ruthenium complex undergoes a redox reaction represented by the following equation.

[Chem. 1] *

In some embodiments, a cation other than ruthenium complexes may be used as the mediator. For example, an osmium complex may be used.

Fig. 1B illustrates a configuration associated with an instance in which a biomolecule as a measurement target is present. The sensor 100 is the same as that of Fig. 1A. When glycated albumin 120, which is a target biomolecule, is introduced into the system, the glycated albumin 120 binds to the aptamer 102. The glycated albumin 120 has a positive charge at lysine 121 and arginine 122. It is believed that the aptamer 102 electrically binds to these positions.

The environment around the regions of the aptamer 102 to which the glycated albumin 120 is bound is electrically neutral. Accordingly, a cationic mediator 113, which is an introduced cationic mediator, reaches the electrode 101 without reacting with the aptamer 102.

Alternatively, the cationic mediator may be introduced first to create the situation illustrated in Fig. 1A, and thereafter, the glycated albumin 120 may be introduced. The glycated albumin 120 binds to the aptamer 102. As a result, the cationic mediator 111 that are electrically bound to the aptamer 102 is released from the aptamer 102, reaches the electrode 101, and can be detected.

Thus, the quantity of the cationic mediator that can be detected by the electrode 101 varies depending on whether the target biomolecule (glycated albumin 120) is absent (Fig. 1A) or present (Fig. 1B). This system can be described in terms of electrical equivalent circuits as follows: in the situation of Fig. 1A, a resistance (R) is relatively high, and a capacitance (C) is relatively low; and in the situation of Fig. 1B, the resistance (R) is relatively low, and the capacitance (C) is relatively high.

In one theory, it is assumed that anions do not have effects similar to those of cationic mediators. In biosensing, [Fe(CN)₆]⁴⁻, for example, is frequently used as an anionic mediator. An assumption is that anions exert a repulsive force on an aptamer, which is typically negatively charged. However, the present invention should not be construed as being limited to these mechanisms and may be described or construed with a different theory or mechanism.

In some embodiments, the aptamer 102 may be a plurality of molecules of the same type disposed on the electrode 101. The target biomolecule (glycated albumin 120) probabilistically reaches the vicinity of the aptamer 102, depending, for instance, on the flow and diffusion of the solution. Accordingly, in accordance with a concentration of the target biomolecule in the solution, the probability that the target biomolecule binds to the aptamer 102 depends on the concentration in the solution. A ratio of an amount of portions of the aptamer 102 that react with the target biomolecule 120 to the total amount of the aptamer 102 disposed on the electrode 101 depends on the concentration of the target biomolecule 120 present in the solution introduced into the vicinity of the aptamer 102. Accordingly, the electrical signal detected by the electrode 101 depends on the concentration of the target biomolecule 120 in the solution. Depending on the concentration of the target biomolecule 120, which is to be measured, in the solution, the quantity or density of the aptamer 102 on the electrode 101 may be varied, a plurality of electrodes 101 with different quantities of the aptamer 102 may be provided, and/or an area of the electrode 101 may be varied.

The quantity of the cationic mediator that can be detected by the electrode 101 varies depending on the concentration of the target biomolecule (glycated albumin 120). Accordingly, the concentration of glycated albumin 120 recognized by the aptamer 102 can be determined based on the electrical signal detected by the electrode 101.

Since the aptamer 102 specifically binds to the target biomolecule (glycated albumin 120), the aptamer 102 substantially does not react with other biomolecules, that is, foreign materials for the measurement. Fig. 1C illustrates a configuration in which a foreign material 130 is present in the system. Examples of the foreign material 130 include human serum albumin (HSA) and immunoglobulins (e.g., IgGs). The aptamer 102, which specifically binds to the glycated albumin 120, does not recognize the foreign material 130. Accordingly, the cationic mediator 111 is electrically attracted to sites of the aptamer 102 that have an opposite charge. Other portions of the cationic mediator, which are referred to as the cationic mediator 112, reach the electrode 101 without feeling the aptamer 102, which has been rendered electrically neutral. This is similar to the situation illustrated in Fig. 1A. It can be said that, electrically, the system illustrated in Fig. 1C is substantially the same as that of Fig. 1A. That is, in an equivalent circuit of Fig. 1C, the resistance (R) and the capacitance (C) are substantially unchanged from those of Fig. 1A. Accordingly, the electrode 101 detects an electrical signal indicative of the absence of the target biomolecule (glycated albumin 120).

### <Protective Film>

Some foreign materials are electrically active or reach the electrode and can be a source of a noise signal. In some embodiments, a protective film may be provided on the surface of the electrode. This enables, for example and without limitation, an influence of foreign materials on the measurement to be eliminated or reduced. For example and without limitation, the protective film can prevent foreign materials from reaching the electrode or being detected by the electrode, with the functions of the aptamer substantially being retained or not being reduced. The protective film can be formed in various manners or processes. Some illustrative embodiments will be described below.

### <Example-1 of Protective Film>

In the example illustrated in Fig. 2A, a diazonium multi-layer film that serves as a protective film 240 is formed on a surface of an electrode 201. The protective film 240 blocks foreign materials, such as HSA, IgGs, and other biomolecules, from approaching the electrode 201 while permitting the glycated albumin that is a target biomolecule to approach the electrode 201. The protective film 240 illustrated in Fig. 2A may be a diazonium multi-layer film.

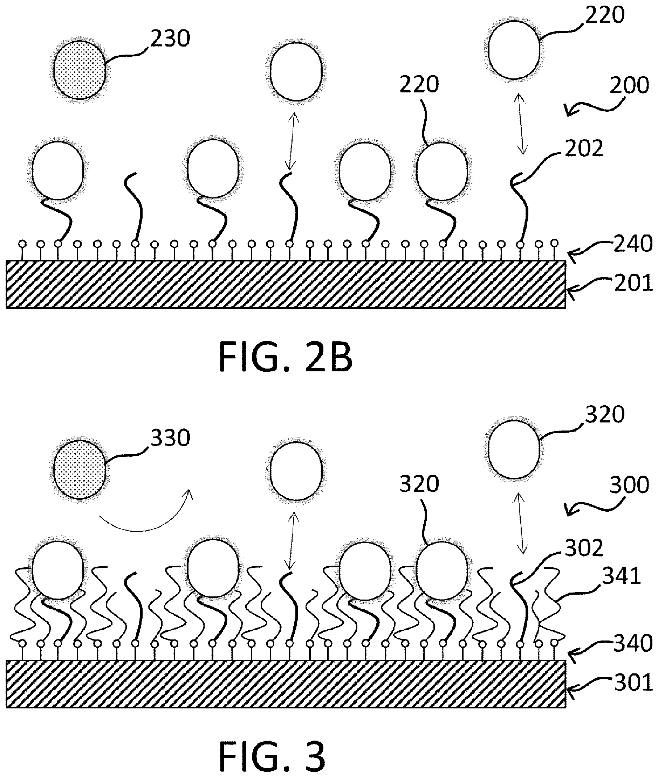

In some embodiments, the sensor may include an electrode, a protective film disposed on the electrode, and an aptamer disposed at a location at which the aptamer can bind to a target biomolecule. Fig. 2B illustrates a sensor 200, which is an example thereof. The sensor 200 includes an electrode 201, a protective film 240, which covers a surface of the electrode 201, and an aptamer 202, which is anchored to a surface of the protective film 240. The protective film 240 also serves as an anchor for anchoring the aptamer 202 to the electrode 201. The protective film 240 can be referred to as an anchor layer. At an end of the anchor layer 240 illustrated in Fig. 2A, the aptamer 202 can be formed (Fig. 2B). The aptamer 202 may be similar to the aptamer 102 illustrated in Figs. 1A to 1C. The aptamer 202 binds to a target biomolecule 220 and does not bind to a foreign material 230. In addition, because of the protective film (anchor layer) 240, the sensor illustrated in Fig. 2B can prevent a reaction of substances that can be sources of noise, such as foreign materials, with the electrode 201. As a result, measurement accuracy can be improved.

### <Example-2 of Protective Film>

In some embodiments, the protective film may have anti-fouling properties (ability to inhibit fouling). In some embodiments, the protective film may contain a polyethylene glycol (PEG). Typically, PEGs have anti-fouling properties. In some embodiments, the PEG may be directly grown or disposed on the electrode. In some embodiments, the PEG (second protective film) may be combined with the anchor layer (first protective film) formed on the electrode.

A sensor 300, illustrated in Fig. 3, is one in which a first protective film (anchor layer) 340 is formed on an electrode 301, and an aptamer 302 and a second protective film (PEG, anti-fouling layer) 341 are formed on the first protective film 340. The PEG is absent on the portions of the surface of the anchor layer 340 in which the aptamer 302 is present. Accordingly, a target biomolecule 320 can bind to the aptamer 302.

For example and without limitation, the PEG may be a PEG (for example, having a molecular weight of 800, 2000, or the like). The first protective film (anchor layer) 340 illustrated in Fig. 3 may be similar to the anchor layer 240 illustrated in Figs. 2A and 2B. In addition, because of the anti-fouling layer 341, the sensor illustrated in Fig. 3 can prevent a reaction of substances that can be sources of noise, such as a foreign material 330, with the electrode 301. As a result, measurement accuracy can be further improved.

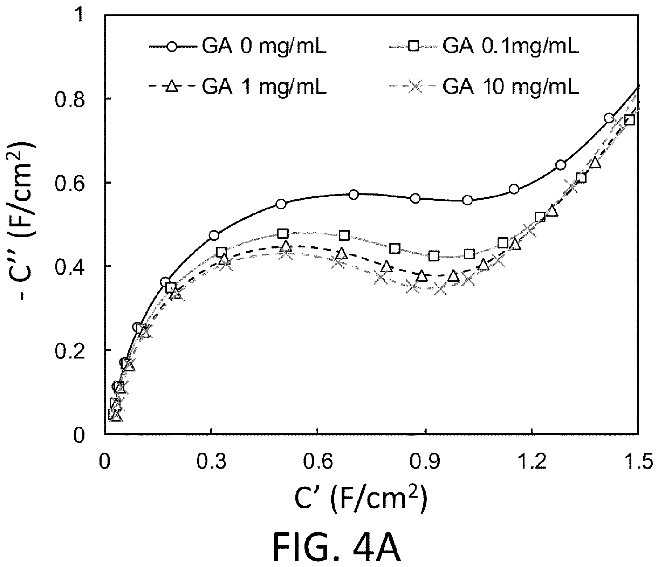

Fig. 4A illustratively shows measurement results obtained with a sensor in which only an anchor layer is disposed on the electrode with no aptamer being present.

Typically, in Nyquist plots of impedance spectra, the vertical axis is an imaginary axis (Z"), and the horizontal axis is a real axis (Z'). A charge transfer resistance Rct can be determined from the diameter of the semicircle of the Nyquist plot on the horizontal axis. However, in the experiments described in Examples herein, it was difficult to present a semicircle because the value R was excessively high, which is probably because of the provision of the anchor layer. Since the anchor layer can be considered in a sense to be an insulating film, the evaluation was performed by using C components. That is, the vertical axis is an imaginary axis (C") of the capacitance, and the horizontal axis is a real axis (C') of the capacitance. Their relationship with the impedance can be expressed as C" (ω) = Z'/ωZ², and C' (ω) = Z"/ωZ². Herein, ω is an angular frequency, and its relationship with a frequency can be expressed as ω = 2πf. The same notations are employed for the other Nyquist plots.

In some embodiments, the impedance measurement may include scanning frequencies. A concentration may be determined from a property of the Nyquist plot, such as a diameter D of the semicircle. For example, the concentration may be determined based on an amount of change (D - D₀) from a diameter D₀, which is a diameter at which the concentration of the target biomolecule is zero. For example, the concentration may be determined based on a ratio (D - D₀)/D₀, which is a ratio of the amount of change (D - D₀) to the diameter D₀ at which the concentration is zero. A calibration curve of the concentration versus a property of the Nyquist plot may be prepared in advance. In some embodiments, the impedance measurement may be performed at multiple frequencies or a single frequency. For example, a calibration curve may be generated by determining in advance a relationship between predetermined frequencies and concentrations. In the actual measurement, the concentration may be determined based on the measurement results obtained with one or more predetermined frequencies.

An EIS measurement was performed by introducing a solution of glycated albumin (GA) into the sensor. The output of the electrode changed with changes in the concentration of the GA, as shown in Fig. 4A. That is, while the anchor layer in the sensor is densely formed, Ru complex ions that serve as the mediator can pass through the anchor layer. The GA non-specifically adsorbs to the surface of the anchor layer, thereby preventing the mediator from passing into the surface of the electrode. That is, the capacitance decreases with an increase in the GA concentration. Signals in a situation in which no aptamers were disposed were measured from the standpoint of a comparison with the following case.

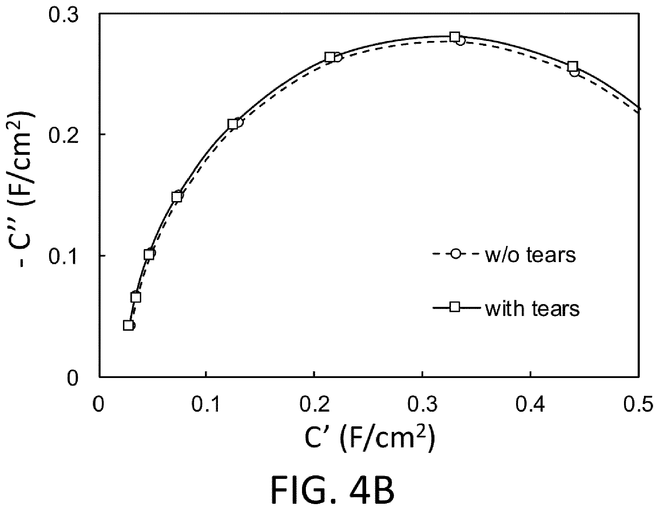

Fig. 4B illustratively shows measurement results obtained with a sensor including a PEG layer. An anchor layer similar to that of Fig. 4A was formed on an electrode, which was different from the electrode of the sensor of Fig. 4A, and the PEG layer was formed on the anchor layer. Thus, the anchor layers of Figs. 4A and 4B can be regarded as being substantially the same. In this instance, no aptamer was formed. Tears (with tears) and a PBS solution (w/o tears) were each introduced to the electrode, and an EIS measurement was performed. The tears contained proteins such as albumin and glycated albumin. The PBS solution did not contain these proteins. As shown in Fig. 4B, no difference was observed in the output of the electrode between the tears and the PBS.

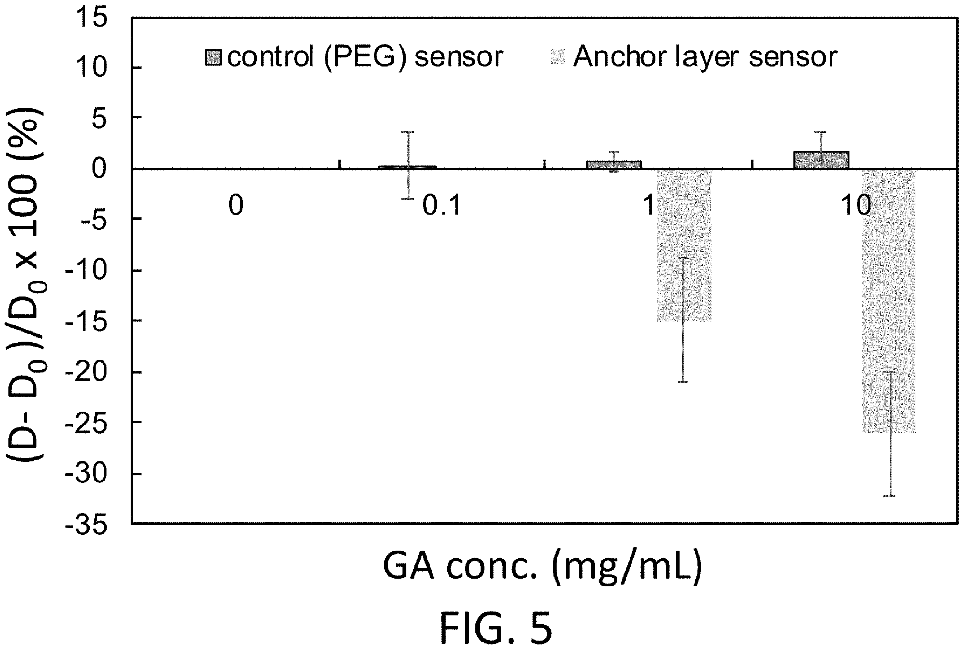

Fig. 5 shows a relationship between the concentration of GA in the solution (horizontal axis) and (D - D₀)/D₀ (vertical axis), based on the measurement results shown in Figs. 4A and 4B. In the instance where the anchor layer was solely present, the value of (D - D₀)/D₀ changed by 1 mg/mL. In the instance where a PEG layer was additionally present, it is apparent that the value substantially did not change for any concentration. Accordingly, the result indicates that foreign materials such as proteins in the tears were blocked or repelled by the PEG layer and, therefore, did not reach the electrode. Thus, it was confirmed that the anti-fouling layer (PEG, in this example) has a high ability to block proteins that are foreign materials.

### <Example-3 of Protective Film>

In some embodiments, the protective film may include bovine serum albumin (BSA). Typically, BSAs have anti-fouling properties. In some embodiments, the BSA may be directly grown or disposed on the electrode. In some embodiments, the BSA (second protective film) may be combined with the anchor layer (first protective film) formed on the electrode. The BSA may be anchored to the anchor layer or need not be anchored to the anchor layer. The BSA molecules may be disposed between portions of an aptamer 402. Accordingly, a target biomolecule 420 can bind to the aptamer 402.

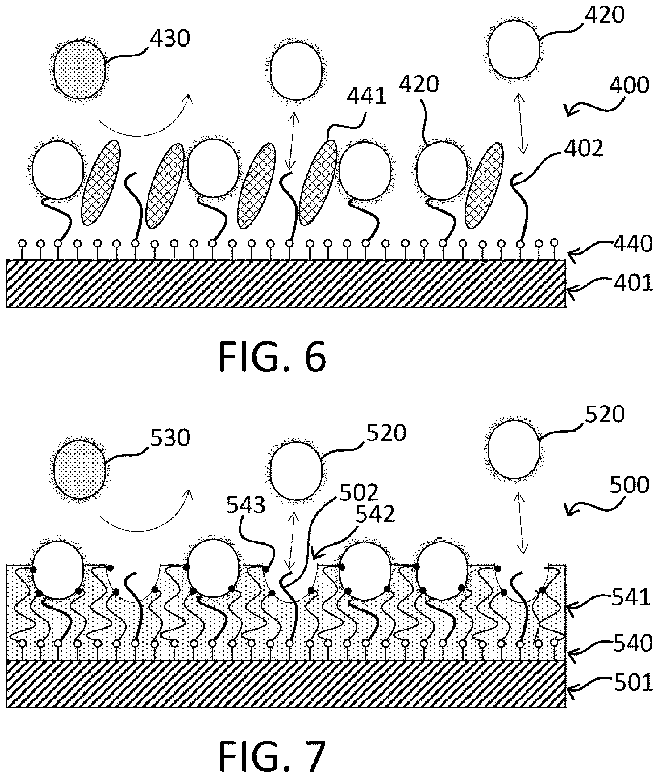

A sensor 400, illustrated in Fig. 6, is one in which a first protective film (anchor layer) 440 is formed on an electrode 401, and an aptamer 402 and a second protective film (BSA, anti-fouling layer) 441 are formed on the first protective film 440. The first protective film (anchor layer) 440 illustrated in Fig. 6 may be similar to the anchor layer 240 illustrated in Figs. 2A and 2B. In addition, because of the BSA layer 441, the sensor 400 illustrated in Fig. 6 can prevent a reaction of substances that can be sources of noise, such as a foreign material 430, with the electrode 401. As a result, measurement accuracy can be further improved.

### <Example-4 of Protective Film>

In some embodiments, the protective film may include a molecularly imprinted polymer (MIP). In some embodiments, the MIP may be directly grown or disposed on the electrode. In some embodiments, the MIP may be combined with the anchor layer (first protective film) formed on the electrode.

A sensor 500, illustrated in Fig. 7, is one in which a first protective film (anchor layer) 540 is formed on an electrode 501, and an MIP layer 541 is formed on the first protective film 540. The first protective film (anchor layer) 540 illustrated in Fig. 7 may be similar to the anchor layer 240 illustrated in Figs. 2A and 2B. For example and without limitation, the MIP layer 541 may be formed by polymerizing a monomer of 2-(methacryloyloxy)ethyl 2-(trimethylammonio)ethyl phosphate. Accordingly, a layer of 2-methacryloyloxyethyl phosphorylcholine (MPC) polymer may be formed.

An end of an aptamer 542 is exposed within a portion (MIP) 542 of the molecularly imprinted polymer of the MIP layer 541. In some embodiments, an MCP polymer that forms the MIP layer 541 may have an end portion exposed within the MIP 542, and a functional group that recognizes a target biomolecule may be disposed at the end portion, as illustrated in Fig. 7. The functional group may be a phenylboronic acid (PBA).

Because of the MIP 542, the aptamer 502 located inside the MIP 542, and a PBA 543, the sensor 500 illustrated in Fig. 7 can recognize more specifically a target biomolecule 520 and prevent a reaction of other substances that can be sources of noise, such as a foreign material 530, with the electrode 501, while the sensor inhibits non-specific adsorption of foreign materials by means of the MIP 542 and the MIP layer 541. As a result, measurement accuracy can be further improved.

### <Example 1: GA sensor>

Regarding several proteins, the output of the electrode was investigated with a sensor having the structure illustrated in Fig. 3. The sensor used includes an electrode and an anchor layer (first protective layer) disposed on the electrode and formed of a diazonium multi-layer film and includes an aptamer for GA and a PEG layer (anti-fouling layer, second protective layer) that are disposed on the anchor layer.

In this example, the sensor used was a GA sensor. The sensor was one in which the electrode was an Au electrode, the first protective film was the diazonium multi-layer film, the second protective layer (anti-fouling layer) was the PEG layer, and the aptamer was a nucleic acid ligand that had a sequence of SEQ ID NO:1 (Table 1) and could specifically bind to GA.

The procedure for the measurement was as follows.
1) First, phosphate buffered saline (PBS) solutions each containing a protein was prepared in the following concentrations. The proteins were GA, HSA, and IgG.
   GA: 0, 0.01, 0.1, 1 mg/mL
   HSA: 0, 0.01, 0.1, 1 mg/mL
   IgG: 0, 0.01, 0.1, 1 mg/mL
   Note that the concentration "0 mg/mL" for GA, HSA, and IgG means that the PBS solution used contained no protein.
2) The proteins at the respective concentrations were each introduced into the sensor. At this stage, GA bound to the aptamer, whereas the other proteins did not bind to the aptamer.
3) Next, B/F (Bond/Free) separation was performed. For example, SDS (sodium dodecyl sulfate) may be used for washing. This enables the washing away, from the sensor, of the substances that did not bind to the aptamer or of the substances that non-specifically adsorbed to the surface of the sensor, for example, the surface of the PEG layer. The proteins other than the GA, which are foreign materials, portions of the GA that did not bind to the aptamer, and the like, for example, do not contribute to the desired measurement and can cause noise. These sources of noise can be removed.
4) Subsequently, a ruthenium complex ([Ru(NH₃)₆]²⁺) was introduced, and the dependence of the output of the electrode on the concentration of the protein was analyzed by EIS.

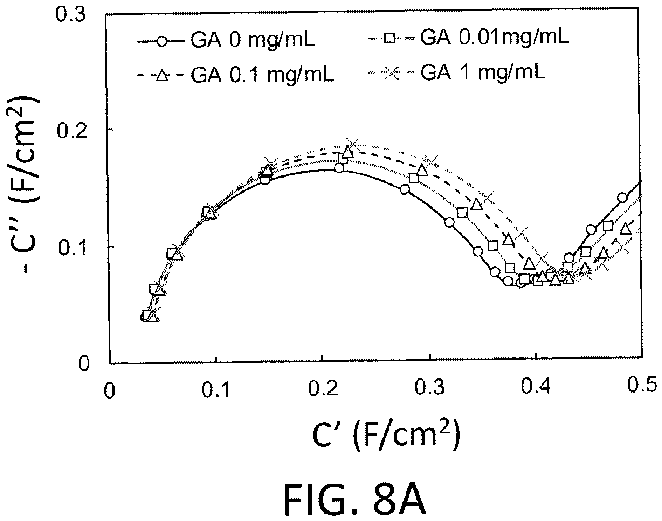

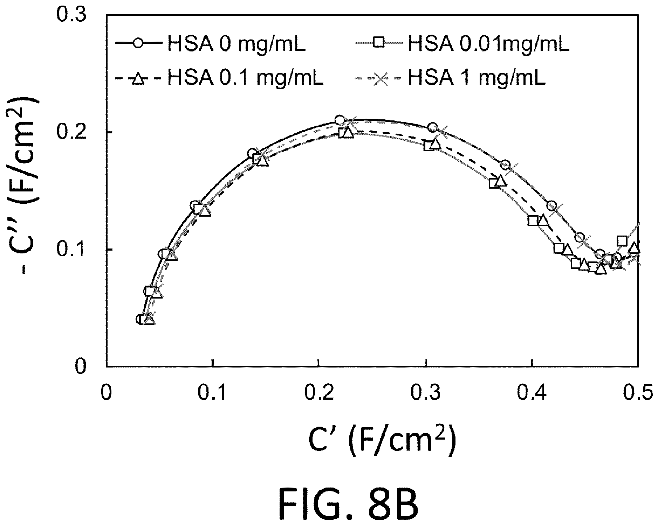

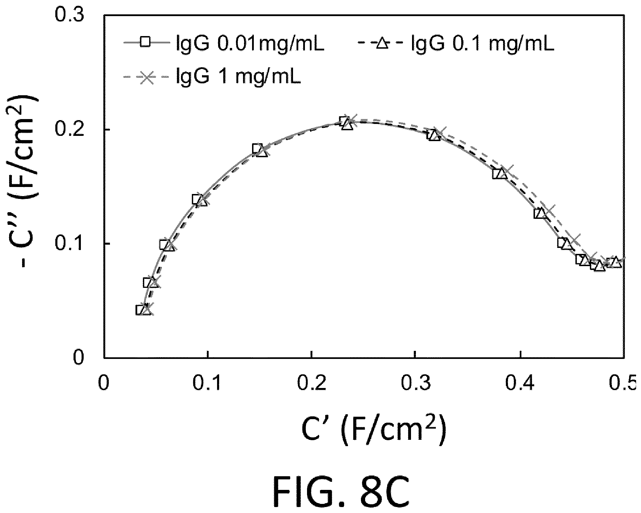

Figs. 8A to 8C show Nyquist plots of the respective proteins regarding multiple concentrations. It was observed that in the case of GA, the plots significantly varied with the concentration, as shown in Fig. 8A. On the other hand, in the case of HSA, the plots slightly varied but did not significantly vary, as shown in Fig. 8B. In the case of IgG, the plots substantially did not vary, as shown in Fig. 8C.

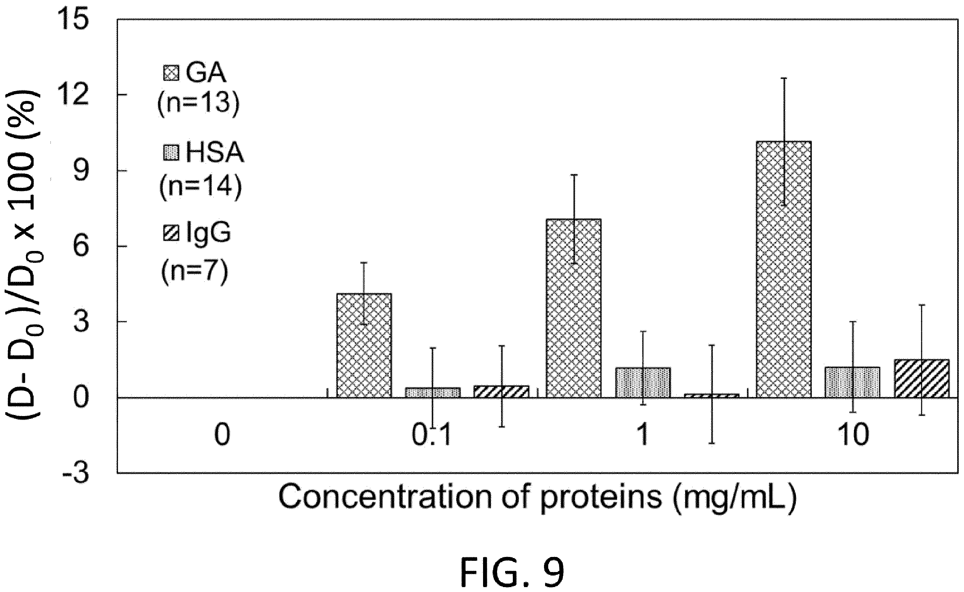

Fig. 9 shows relationships between the concentration of each of the proteins (horizontal axis) and the amount of change (D - D₀) /D₀ in the diameter of the Nyquist plots (vertical axis) based on the measurement results shown in Figs. 8A to 8C. It was found that in the case of GA, the value of (D - D₀) /D₀ was dependent on the concentration, whereas in the case of HSA and IgG, the value substantially was not dependent on the concentration or was dependent on the concentration to a much lower degree than in the case of GA. Accordingly, with this configuration, it is possible to produce a sensor for specifically recognizing GA or measuring the concentration of GA.

### <Albumin Sensor>

The present disclosure also provides an albumin sensor. In some embodiments, the albumin sensor includes an electrode and an albumin antibody (HSA antibody or anti-albumin antibody) disposed on or near the electrode.

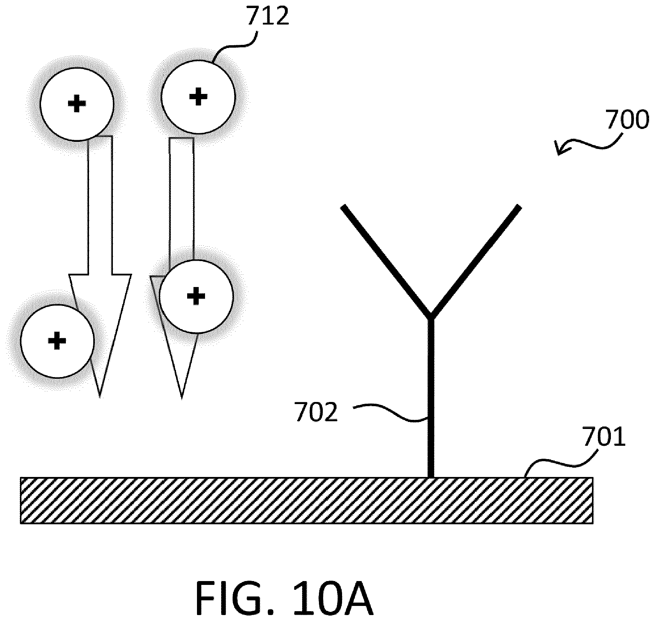

Fig. 10A illustrates an albumin sensor 700, according to an embodiment. The albumin sensor 700 includes an electrode 701 and an albumin antibody (HSA antibody) 702, which is disposed on the electrode 701. The albumin sensor 700 can detect or measure an introduced mediator 712 at the electrode 701.

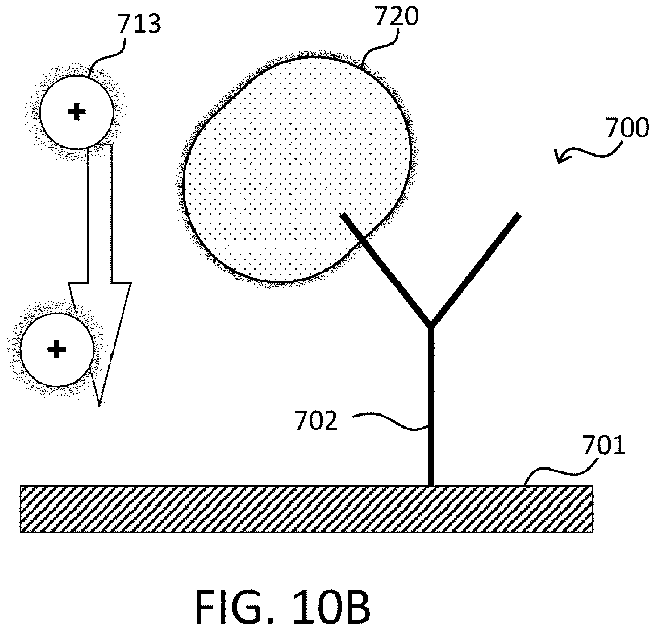

When albumin 720, which is a target biomolecule, is introduced, the albumin 720 is recognized and captured by the albumin antibody 720 (Fig. 10B). The captured albumin 720 reduces an effective surface area of the electrode 701, thereby limiting access of the mediator 712 to the electrode 701. This system can be described in terms of electrical equivalent circuits as follows: in the situation of Fig. 10B, the resistance (R) is high, and the capacitance (C) is low, with respect to the situation of Fig. 10A.

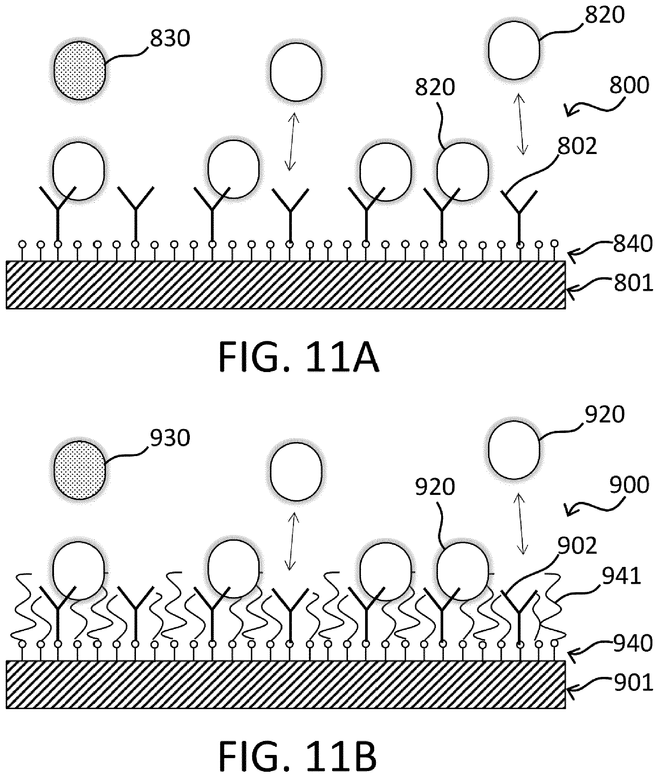

Fig. 11A illustrates a sensor 800, according to an example. The sensor 800 includes an electrode 801, a protective film 840, which covers a surface of the electrode 801, and an antibody 802, which is anchored to a surface of the protective film 840. For example and without limitation, the protective film 840 may be similar to the anchor layer 240 illustrated in Fig. 2B. The antibody 802 may be formed at an end of the protective film (anchor layer) 840, as in Fig. 2B. The antibody 802 binds to albumin 820, which is a target biomolecule, and does not bind to a foreign material 830. In addition, because of the protective layer 840, the sensor 800 illustrated in Fig. 11A can prevent a reaction of substances that can be sources of noise, such as foreign materials, with the electrode 801. As a result, measurement accuracy can be improved.

Fig. 11B illustrates a sensor 900, according to an example. The sensor 900 is one in which a first protective film (anchor layer) 940 is formed on an electrode 901, and an antibody 902 and a second protective film (PEG, anti-fouling layer) 941 are formed on the first protective film 940. The PEG is absent on the portions of the surface of the anchor layer 340 in which the antibody 902 is present. Accordingly, a target biomolecule 920 can bind to the antibody 902.

For example and without limitation, the PEG may be PEG-800 or PEG-2000. The first protective film (anchor layer) 940 illustrated in Fig. 11B may be similar to the anchor layer 240 illustrated in Figs. 2A and 2B. In addition, because of the anti-fouling layer 941, the sensor 900 illustrated in Fig. 11B can prevent a reaction of substances that can be sources of noise, such as a foreign material 930, with the electrode 901. As a result, measurement accuracy can be further improved.

### <Example: HSA sensor>

Regarding GA and HSA, the output of the electrode was investigated with a sensor having the structure illustrated in Fig. 11B. The sensor used includes an electrode and an anchor layer (first protective layer) disposed on the electrode and formed of a diazonium multi-layer film and includes an anti-albumin antibody and a PEG layer (anti-fouling layer, second protective layer) that are disposed on the anchor layer.

The procedure for the measurement was as follows.
1) First, phosphate buffered saline (PBS) solutions each containing a protein was prepared in the following concentrations. The proteins were HSA and GA.
   HSA: 0, 0.1, 1, 10 mg/mL
   GA: 0, 0.1, 1, 10 mg/mL
   Note that the concentration "0 mg/mL" for HSA and GA means that the PBS solution used contained no protein.
2) The proteins at the respective concentrations were each introduced into the sensor and sufficiently reacted with the antibody.
3) Next, B/F (Bond/Free) separation was performed.
   For example, SDS (sodium dodecyl sulfate) may be used for washing. This enables the washing away, from the sensor, of the substances that did not bind to the aptamer or of the substances that non-specifically adsorbed to the surface of the sensor, for example, the surface of the PEG layer.
4) Subsequently, a ruthenium complex ([Ru(NH₃)₆]²⁺) was introduced, and the dependence of the output of the electrode on the concentration of the protein was analyzed by EIS.

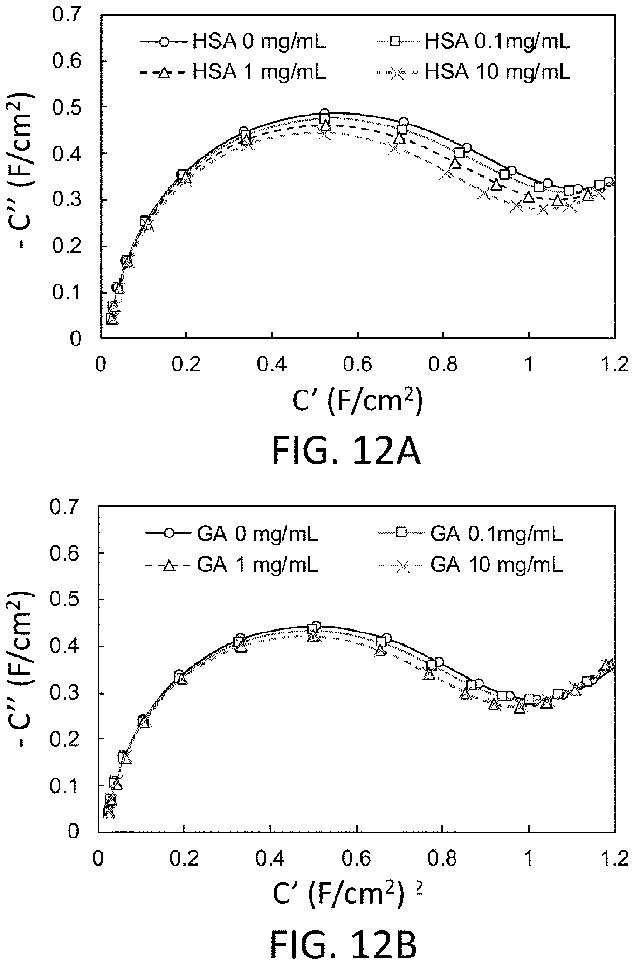

Figs. 12A to 12B show Nyquist plots of the HSA and the GA regarding multiple concentrations. It was observed that in the case of HSA, the plots varied with the concentration, as shown in Fig. 12A. In the case of GA, the plots varied although the degree of variation was not as high as that in Fig. 12A, as shown in Fig. 12B. Presumably, the reason that the output from the HSA antibody was affected by the GA was the non-specific adsorption of the GA to the HSA antibody. However, there is a study regarding whether or not there is a possibility that the HSA antibody used in this Example might also recognize GA.

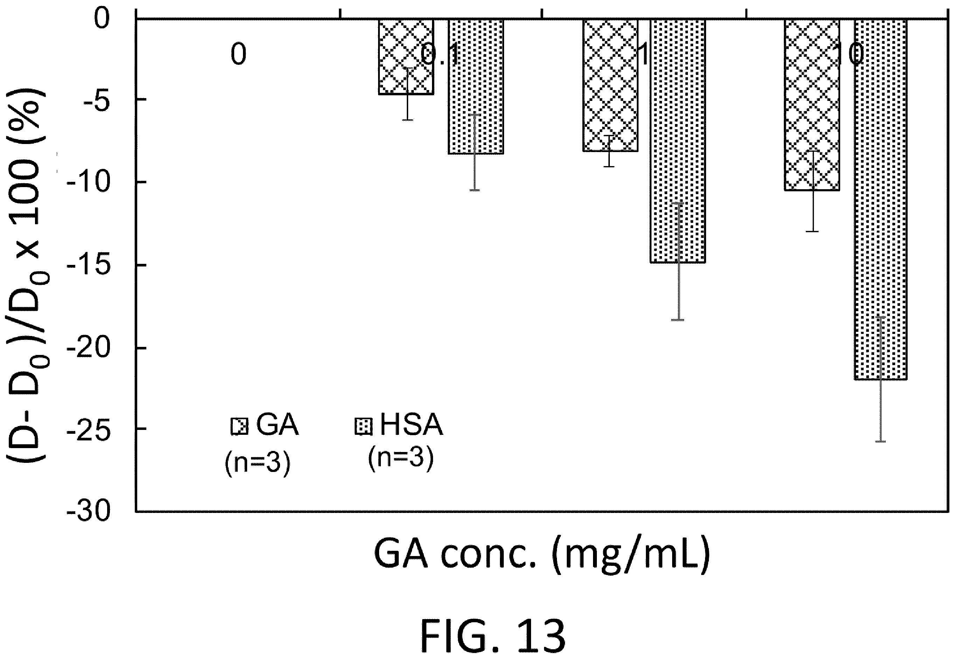

Fig. 13 shows relationships between the concentration of each of the proteins (horizontal axis) and the amount of change (D - D₀) /D₀ in the diameter of the Nyquist plots (vertical axis) based on the measurement results shown in Figs. 12A and 12B. It was found that in the case of HSA, the value of (D - D₀) /D₀ was dependent on the concentration. Accordingly, with this configuration, it is possible to produce a sensor for measuring the concentration of albumin in a solution.

The influence of GA observed in Figs. 12B and 13 can be removed or corrected. For example, an antibody that specifically recognizes HSA (or non-glycated albumin) and does not recognize GA may be used. In this instance, a GA value can be determined as a ratio ([GA]/{[HSA]+[GA]}), which is a ratio of the concentration (numerator) ([GA]) of glycated albumin to the sum (denominator) of the concentration ([HSA]) of non-glycated albumin, which is determined using the HSA antibody, and the concentration ([GA]) of glycated albumin, which is determined using the GA aptamer. In the instance where the HSA antibody recognizes GA to some extent, the output of the sensor having the HSA antibody equals the sum ([HSA]+α[GA]) of the concentration of non-glycated albumin and a portion of the concentration of glycated albumin. Accordingly, the actual concentration of HSA can be determined by determining the ratio α. It is believed that a specific or non-specific influence of other substances, such as an IgG, can also be removed or corrected in a similar manner or with a different technique.

A calibration curve can be generated based on, for instance, the data shown in Fig. 9. The concentration of GA and the concentration of albumin can be determined from the calibration curve. The GA value can be determined as a ratio of the concentration of GA to the concentration of albumin.

Note that the manner in which the GA value is determined is not limited to the above-described manner. Typically, the GA value is obtained by dividing an amount of glycated albumin by a total amount of non-glycated albumin and glycated albumin. In some embodiments, the GA value may be determined based on the concentration of non-glycated albumin (HSA) and a concentration of GA determined with a different sensor. In some embodiments, the GA value may be determined based on a total value of the concentration of non-glycated albumin (HSA) and the concentration of glycated albumin (GA) or a value calculated from these and on a concentration of GA determined with a different sensor.

### <Dual Sensor>

A ratio of the concentration of GA to the concentration of HSA, which is a so-called GA value, can be determined by using a GA sensor and an albumin sensor. In some embodiments, an apparatus for measuring the GA value may include a GA sensor and an albumin sensor. In some embodiments, the GA sensor and the albumin sensor may be configured to receive the same measurement target solution and perform a measurement. For example, both of the sensors may be disposed within a container, flow passage, or volume for the measurement target solution. The apparatus may be configured such that, for example, the GA sensor and the albumin sensor are disposed within different respective containers, into which a measurement target solution can be introduced in portions.

The albumin sensor is a sensor for measuring the total amount of glycated albumin and non-glycated albumin or the amount of non-glycated albumin. The form of the albumin sensor for use in the dual sensor of the present disclosure should not be particularly limited.

In some embodiments, an apparatus for measuring the GA value may include a GA sensor and an albumin sensor. In some embodiments, a system or a unit for measuring the GA value may be configured to be connected to a GA sensor and an albumin sensor.

### <EIS and Nyquist Plot>

EIS was performed as follows. An impedance measurement was performed at various frequencies by scanning frequencies of 100 to 1,000,000 Hz (1 MHz). An electrochemical analyzer from BAS was used for the measurement. An initial voltage was - 0.14 V. A phosphate buffer solution (pH: 7.4) containing Ru complex ions as the mediator was used. For the measurement of the reaction between the electrode and a protein such as GA, a phosphate buffer solution (pH: 7.4) was used, with no Ru complex ions added thereto. After the protein was reacted with the aptamer, washing was performed, that is, B/F separation was performed, and thereafter, the measurement was performed.

### <Measurement Method: Example 1>

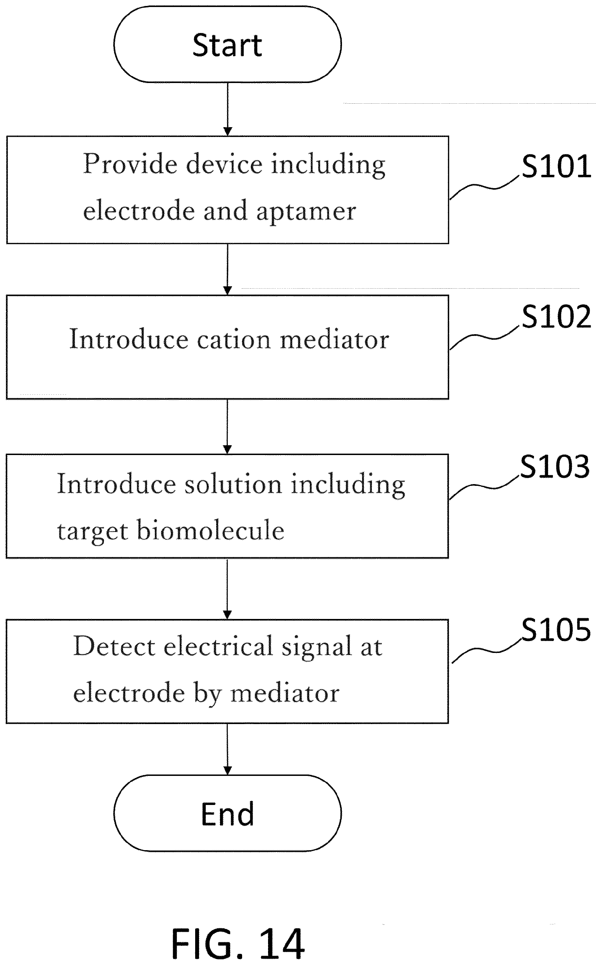

Fig. 14 illustrates a process of a method for measuring a biomolecule according to an embodiment. At step S101, a device or an apparatus is provided or prepared. The device or apparatus includes an electrode and an aptamer that is disposed near the electrode and which specifically binds to a target biomolecule. At step S102, a cationic mediator is introduced into the system. The cationic mediator binds to the sites of the aptamer that have an opposite charge. At step S103, a target biomolecule is introduced. Alternatively, a solution that contains or may contain the target biomolecule may be introduced into the system. At step S105, an electrical signal at the electrode is read. The electrical signal is produced by a reaction (e.g., a redox reaction) between the cationic mediator and the electrode. Accordingly, an amount of portions of the target biomolecule that are bound to the aptamer is quantified. An example of the amount is a concentration of the target biomolecule in the introduced solution.

### <Measurement Method: Example 2>

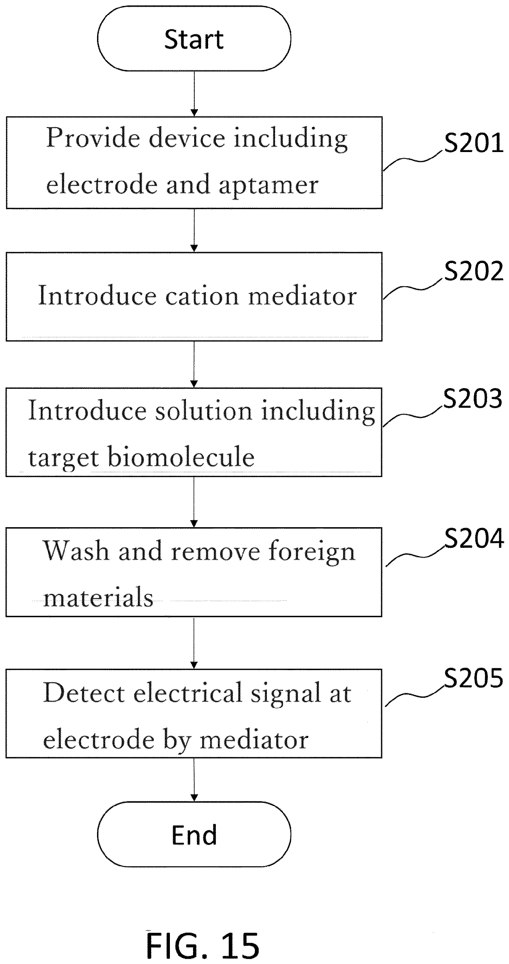

Fig. 15 illustrates a process of a method for measuring a biomolecule according to an embodiment. Steps S201 to S203 are the same as, or the same at least in terms of a purpose as, steps S101 to S103. At step S204, foreign materials present in the system may be removed. This step may be referred to as washing. This step may include B/F separation. At step S205, an electrical signal at the electrode is read. The electrical signal is produced by a reaction (e.g., a redox reaction) between the cationic mediator and the electrode. Accordingly, an amount of portions of the target biomolecule that are bound to the aptamer is quantified. An example of the amount is a concentration of the target biomolecule in the introduced solution. In the instance where washing is performed at step S204 to remove or reduce foreign materials that can affect the measurement, the measurement can be performed more accurately.

The present disclosure also provides the following embodiments.

### A001

A method for detecting a biomolecule, comprising:
providing an electrode and an aptamer that specifically binds to a target biomolecule, the aptamer being disposed near the electrode or anchored to the electrode;
introducing a cationic mediator to the electrode at which the aptamer is disposed;
bringing a solution containing the target biomolecule into contact with the aptamer to cause the aptamer to bind to the biomolecule; and
measuring an electrical signal that is produced at the electrode in association with the cationic mediator.

### A001b

The method according to embodiment A001 or any embodiment, wherein the mediator is a cationic mediator.

### A001c

A method for detecting a biomolecule, comprising:
providing an electrode and an aptamer that specifically binds to a target biomolecule and has a charge, the aptamer being disposed near the electrode or anchored to the electrode;
introducing a mediator having a charge opposite to the charge of the aptamer to the electrode at which the aptamer is disposed;
bringing a solution containing the target biomolecule into contact with the aptamer to cause the aptamer to bind to the biomolecule; and
measuring an electrical signal that is produced at the electrode in association with the mediator.

### A002

The method according to any one of embodiments A001 to A001c or any embodiment, further comprising determining an amount of the target biomolecule present in the solution, based on the electrical signal.

### A002b

The method according to embodiment A002 or any embodiment, wherein the amount of the target biomolecule present in the solution is determined based on a capacitance component of the electrical signal.

### A002c

The method according to embodiment A002 or any embodiment, wherein the amount of the target biomolecule present in the solution is determined based on a current component of the electrical signal.

### A003

The method according to any one of embodiments A001 to A002c or any embodiment, wherein the measuring of the electrical signal that is produced at the electrode in association with the mediator comprises performing electrochemical impedance spectroscopy.

### A004

The method according to embodiment A003 or any embodiment, further comprising evaluating a capacitance component of a redox reaction of the mediator that occurs at the electrode, the capacitance component being a capacitance component obtained from the electrochemical impedance spectroscopy.

### A004b

The method according to embodiment A003 or any embodiment, further comprising evaluating a value associated with a charge transfer resistance (Rct) of a redox reaction of the mediator that occurs at the electrode, the value being a value obtained from the electrochemical impedance spectroscopy.

### A005

The method according to embodiment A004 or any embodiment, wherein an amount of portions of the target biomolecule that are bound to the aptamer is determined based on a value of the capacitance component of the redox reaction of the mediator that occurs at the electrode.

### A005b

The method according to embodiment A004 or any embodiment, wherein an amount of portions of the target biomolecule that are bound to the aptamer is determined based on the value associated with the charge transfer resistance (Rct) of the redox reaction of the mediator that occurs at the electrode.

### A011

The method according to any one of embodiments A001 to A005b or any embodiment, wherein the target biomolecule is a glycated protein.

### A012

The method according to embodiment A011 or any embodiment, wherein the target biomolecule is glycated albumin.

### A013

The method according to any one of embodiments A001 to A005b or any embodiment, wherein the target biomolecule is one or more selected from the group consisting of cells, viruses, and extracellular vesicles.

### A021

The method according to embodiment A012 or any embodiment, wherein the aptamer comprises a nucleotide sequence of SEQ ID NO:1.

### A031

The method according to any one of embodiments A001 to A021 or any embodiment, wherein the mediator is a ruthenium complex.

### A041

The method according to any one of embodiments A001 to A031 or any embodiment, wherein the introducing of the mediator to the electrode at which the aptamer is disposed is performed before the bringing of the biomolecule into contact with the aptamer.

### A042

The method according to embodiment A041 or any embodiment, further comprising substantially removing portions of the mediator that are not bound to the aptamer, the removing being performed after the introducing of the mediator to the electrode at which the aptamer is disposed and before the bringing of the biomolecule into contact with the aptamer.

### A051

The method according to any one of embodiments A001 to A042 or any embodiment, wherein the measuring of the electrical signal that is produced at the electrode in association with the mediator comprises performing an impedance measurement.

### B001

A method for evaluating a degree of glycation of a protein, comprising:
providing an electrode that has an aptamer disposed on a surface of the electrode, the aptamer being an aptamer that specifically binds to a glycated form of a target protein;
introducing a solution containing the glycated form of the target protein and a non-glycated form of the target protein to the electrode to cause the glycated form of the target protein to bind to the aptamer;
introducing a mediator to the electrode at which the aptamer is disposed;
measuring an electrical signal that is produced at the electrode in association with the mediator;
determining an amount of the glycated form of the target protein present in the solution, based on the measured electrical signal;
determining an amount of the non-glycated form of the target protein present in the solution or a total amount of the glycated form of the target protein and the non-glycated form of the target protein that are present in the solution; and
determining the degree of glycation of the target protein based on the amount of the glycated form of the target protein and on the amount of the non-glycated form of the target protein or the total amount of the glycated form of the target protein and the non-glycated form of the target protein.

### B011

A method for evaluating a degree of glycation of a protein, comprising:
providing a first electrode that has an aptamer disposed on a surface of the first electrode, the aptamer being an aptamer that specifically binds to a glycated target protein;
providing a second electrode that has an antibody disposed on a surface of the second electrode, the antibody being an antibody that recognizes a second target protein;
introducing a solution containing the glycated target protein and the second target protein to the first electrode and the second electrode to cause the glycated target protein to bind to the aptamer and cause the second target protein to bind to the antibody;
introducing a mediator to the first electrode at which the aptamer is disposed and to the second electrode;
measuring a first electrical signal that is produced at the first electrode in association with the mediator and measuring a second electrical signal that is produced at the second electrode; and
determining a ratio of the glycated target protein present in the solution to the second target protein present in the solution, based on the measured first electrical signal and the measured second electrical signal.

### C001

An apparatus for detecting a biomolecule, comprising:
an electrode; and
an aptamer that specifically binds to a target biomolecule, the aptamer being disposed on a surface of the electrode,
the apparatus being configured to
allow a solution containing the target biomolecule to come into contact with the aptamer;
allow a mediator to be introduced to the electrode at which the aptamer is disposed; and
measure an electrical signal that is produced at the electrode in association with the mediator.

### C002

The apparatus according to embodiment C001 or any embodiment, wherein the mediator is a cationic mediator.

### C011

The apparatus according to embodiment C001 or any embodiment, further comprising a protective film that is disposed on the surface of the electrode and has an ability to allow passage of the mediator toward the electrode through the protective film and to inhibit passage of a foreign material toward the electrode through the protective film.

### C012

The apparatus according to embodiment C011 or any embodiment, wherein the protective film comprises a diazonium multi-layer film.

### C013

The apparatus according to embodiment C012 or any embodiment, wherein the aptamer is anchored to the diazonium multi-layer film.

### C014

The apparatus according to any one of embodiments C011 to C013 or any embodiment, wherein the protective film comprises a second protective film having an anti-fouling property.

### C015

The apparatus according to embodiment C012 or any embodiment, wherein the protective film comprises a second protective film having an anti-fouling property, the second protective film being formed on a surface of the diazonium multi-layer film.

### C016

The apparatus according to embodiment C014 or C015 or any embodiment, wherein the second protective film consists essentially of a polyethylene glycol (PEG) or bovine serum albumin (BSA).

### C021

The apparatus according to any one of embodiments C011 to C013 or any embodiment, wherein the protective film comprises a molecularly imprinted polymer (MIP) that recognizes the target biomolecule and within which an end of the aptamer is exposed.

### C022

The apparatus according to embodiment C021 or any embodiment, wherein the molecularly imprinted polymer comprises a functional group that is disposed on a surface within the molecularly imprinted polymer and recognizes the target biomolecule.

### C023

The apparatus according to embodiment C021 or any embodiment, wherein the functional group is phenylboronic acid (PBA).

### C031

The apparatus according to any one of embodiments C001 to C023 or any embodiment, further comprising a measurement device connected to the electrode.

### C032

The apparatus according to any one of embodiments C011 to C023 or any embodiment, wherein the measurement device is capable of performing a measurement that uses electrochemical impedance spectroscopy.

### C101

A device for measuring glycated albumin, comprising:
an electrode; and
an aptamer disposed on a surface of the electrode and comprising a sequence of SEQ ID NO:1.

### D001

An apparatus for determining a GA value, comprising:
the device for measuring glycated albumin according to embodiment C101 or any embodiment; and
a device for measuring albumin.

### D002

The apparatus according to embodiment D001 or any embodiment, wherein the device for measuring albumin comprises an electrode and an anti-albumin antibody disposed on a surface of the electrode.

While several embodiments and examples of the present disclosure have been described above, these embodiments and examples illustratively describe the present disclosure. For example, the above embodiments are those described in detail so that the present disclosure can be clearly described, and, if necessary, additional modifications may be made to dimensions, configurations, materials, and circuits. Note that the scope of the present disclosure also encompasses embodiments that have one or more of the above-described features of the present disclosure in any combination. It is intended that the appended claims cover numerous modifications to the embodiments within the technical spirit of the present disclosure. Accordingly, it is to be understood that the embodiments and examples disclosed herein have been presented by way of illustration and should not be considered as limiting the scope of the present disclosure.

### References

Shoichi Nishitani and *T. Sakata "Polymeric Nanofilter Biointerface for Potentiometric Small-Biomolecule Recognition", ACS Appl. Mater. Interfaces, 11 (5), (2019), pp 5561–5569. (DOI: 10.1021/acsami.8b20010)
Shogo Himori, Shoichi Nishitani and *T. Sakata "Control of Potential Response to Small Biomolecule with Electrochemically Grafted Aryl-Based Monolayer in Field-Effect-Transistor-Based Sensor" Langmuir, 35 (10), (2019), pp 3701–3709. (DOI: 10.1021/acs.langmuir.9b00085)

## Claims

1. A method for detecting a biomolecule, comprising:
providing an electrode and an aptamer that specifically binds to a target biomolecule and has a charge, the aptamer being disposed near the electrode or anchored to the electrode;
introducing a mediator having a charge opposite to the charge of the aptamer to the electrode at which the aptamer is disposed;
bringing a solution containing the target biomolecule into contact with the aptamer to cause the aptamer to bind to the biomolecule; and
measuring an electrical signal that is produced at the electrode in association with the mediator.

2. The method according to Claim 1, further comprising determining an amount of the target biomolecule present in the solution, based on the electrical signal.

3. The method according to Claim 1 or 2, wherein the measuring of the electrical signal that is produced at the electrode in association with the mediator comprises performing electrochemical impedance spectroscopy.

4. The method according to Claim 3, further comprising evaluating a capacitance component of a redox reaction of the mediator that occurs at the electrode, the capacitance component being a capacitance component obtained from the electrochemical impedance spectroscopy.

5. The method according to Claim 4, wherein an amount of portions of the target biomolecule that are bound to the aptamer is determined based on a value of the capacitance component of the redox reaction of the mediator that occurs at the electrode.

6. The method according to Claim 1 or 2, wherein the target biomolecule is a glycated protein.

7. The method according to Claim 6, wherein the target biomolecule is glycated albumin.

8. The method according to Claim 1 or 2, wherein the target biomolecule is one or more selected from the group consisting of cells, viruses, and extracellular vesicles.

9. The method according to Claim 8, wherein the aptamer comprises a nucleotide sequence of SEQ ID NO: 1.

10. The method according to Claim 1, wherein the mediator is a ruthenium complex.

11. The method according to Claim 1 or 10, wherein the introducing of the mediator to the electrode at which the aptamer is disposed is performed before the bringing of the biomolecule into contact with the aptamer.

12. The method according to Claim 11, further comprising substantially removing portions of the cationic mediator that are not bound to the aptamer, the removing being performed after the introducing of the mediator to the electrode at which the aptamer is disposed and before the bringing of the biomolecule into contact with the aptamer.

13. A method for evaluating a degree of glycation of a protein, comprising:
providing an electrode that has an aptamer disposed on a surface of the electrode, the aptamer being an aptamer that specifically binds to a glycated form of a target protein;
introducing a solution containing the glycated form of the target protein and a non-glycated form of the target protein to the electrode to cause the glycated form of the target protein to bind to the aptamer;
introducing a mediator having a charge opposite to the charge of the aptamer to the electrode at which the aptamer is disposed;
measuring an electrical signal that is produced at the electrode in association with the mediator;
determining an amount of the glycated form of the target protein present in the solution, based on the measured electrical signal;
determining an amount of the non-glycated form of the target protein present in the solution or a total amount of the glycated form of the target protein and the non-glycated form of the target protein that are present in the solution; and
determining the degree of glycation of the target protein based on the amount of the glycated form of the target protein and on the amount of the non-glycated form of the target protein or the total amount of the glycated form of the target protein and the non-glycated form of the target protein.

14. A method for evaluating a degree of glycation of a protein, comprising:
providing a first electrode that has an aptamer disposed on a surface of the first electrode, the aptamer being an aptamer that specifically binds to a glycated target protein;
providing a second electrode that has an antibody disposed on a surface of the second electrode, the antibody being an antibody that recognizes a second target protein;
introducing a solution containing the glycated target protein and the second target protein to the first electrode and the second electrode to cause the glycated target protein to bind to the aptamer and cause the second target protein to bind to the antibody;
introducing a mediator having a charge opposite to the charge of the aptamer to the first electrode at which the aptamer is disposed and to the second electrode;
measuring a first electrical signal that is produced at the first electrode in association with the mediator and measuring a second electrical signal that is produced at the second electrode; and
determining a ratio of the glycated target protein present in the solution to the second target protein present in the solution, based on the measured first electrical signal and the measured second electrical signal.

15. An apparatus for detecting a biomolecule, comprising:
an electrode; and
an aptamer that specifically binds to a target biomolecule, the aptamer being disposed on a surface of the electrode,
the apparatus being configured to
allow a solution containing the target biomolecule to come into contact with the aptamer;
allow a cationic mediator to be introduced to the electrode at which the aptamer is disposed; and
measure an electrical signal that is produced at the electrode in association with the mediator.

16. The apparatus according to Claim 15, further comprising a protective film that is disposed on the surface of the electrode and has an ability to allow passage of the mediator toward the electrode through the protective film and to inhibit passage of a foreign material toward the electrode through the protective film.

17. The apparatus according to Claim 16, wherein the protective film comprises a diazonium multi-layer film.

18. The apparatus according to Claim 17, wherein the aptamer is anchored to the diazonium multi-layer film.

19. The apparatus according to Claim 16, wherein the protective film comprises a second protective film having an anti-fouling property.

20. The apparatus according to Claim 19, wherein the protective film comprises a second protective film having an anti-fouling property, the second protective film being formed on a surface of the diazonium multi-layer film.

21. The apparatus according to Claim 19 or 20, wherein the second protective film consists essentially of a polyethylene glycol (PEG) or bovine serum albumin (BSA).

22. The apparatus according to Claim 19 or 20, wherein the protective film comprises a molecularly imprinted polymer (MIP) that recognizes the target biomolecule and within which an end of the aptamer is exposed.

23. The apparatus according to Claim 22, wherein the molecularly imprinted polymer comprises a functional group that is disposed on a surface within the molecularly imprinted polymer and recognizes the target biomolecule.

24. The apparatus according to Claim 15, further comprising a measurement device that is connected to the electrode and performs electrochemical impedance spectroscopy.

25. A device for measuring glycated albumin, comprising:
an electrode; and
an aptamer disposed on a surface of the electrode and comprising a sequence of SEQ ID NO:1.

26. An apparatus for determining a GA value, comprising:
the device for measuring glycated albumin according to Claim 25; and
a device for measuring albumin.

27. The apparatus according to Claim 26, wherein the device for measuring albumin comprises an electrode and an anti-albumin antibody disposed on a surface of the electrode.
